# EUROPEAN PATENT SPECIFICATION

(11) **EP 4 055 413 B1**
(45) Date of publication and mention of the grant of the patent: **11.12.2024**
(21) Application number: 20797765.3
(22) Date of filing: 29.10.2020
(51) Int. Cl.: G01S 15/89, G01S 7/52, A61B 8/12, A61B 8/00

(54) **AN ULTRASOUND IMAGING CATHETER**
KATHETER ZUR ULTRASCHALLBILDGEBUNG
CATHÉTER D'IMAGERIE À ULTRASONS

(30) Priority: 05.11.2019 EP 19207183
(43) Date of publication of application: 14.09.2022
(73) Proprietor: Koninklijke Philips N.V., 5656 AG Eindhoven (NL)
(72) Inventor: DOUGLAS, Alexander Ulrich, 5656 AE Eindhoven (NL); MOUNAIM, Amine, 5656 AE Eindhoven (NL); VAN RENS, Antonia Cornelia, 5656 AE Eindhoven (NL)
(74) Representative: Philips Intellectual Property & Standards
(86) International application number: PCT/EP2020/080335
(87) International publication number: WO 2021/089389

(56) References cited:
- US-A1- 2015 087 991
- WYGANT I ET AL: "P3H-6 An Integrated Circuit with Transmit Beamforming and Parallel Receive Channels for Real-Time Three-Dimensional Ultrasound Imaging", ULTRASONICS SYMPOSIUM, 2006. IEEE, IEEE, PI, 2 October 2006 (2006-10-02), pages 2186 - 2189, XP032392067, ISBN: 978-1-4244-0201-4, DOI: 10.1109/ULTSYM.2006.551

## Description

### FIELD OF THE INVENTION

The invention relates to the field of ultrasound imaging systems, and more specifically to ultrasound imaging catheters.

### BACKGROUND OF THE INVENTION

Intravenous ultrasound imaging catheters are widely used in a number of different clinical applications. Typically, an ultrasound imaging catheter is fully controlled from a back end processing unit, or console. For each ultrasound transducer element in the ultrasound imaging catheter, it needs to be decided whether the transducer element is active during transmission of an ultrasonic pulse and whether the transducer element should be active during the reception of echoes from the transmitted pulse.

In order to improve the imaging quality of ultrasound imaging catheters, attempts have been made to increase the number of transducer elements that can be located at the tip of the catheter.

By increasing the number of transducer elements, and increasing the number of return channels for the echoes, the full control of the array of transducer elements at the catheter tip by the console causes significant challenges, such as steep signal slopes and high bandwidth requirements, resulting in increased strain on the communication between the console and the catheter.

Typically, communication channels in ultrasound imaging catheter systems are comprised of wires having relatively high resistances and capacitances, due to mechanical requirements, causing interference in the communication signals and degrading the accuracy of the images produced by the ultrasound imaging catheter.

US 2015/087991 A1 discloses an ultrasonic imaging system in which a column-row-parallel architecture is provided at the circuit level of an ultrasonic transceiver. The ultrasonic imaging system includes an N×M array of transducer elements and a plurality of transceiver circuits, where each transceiver circuit is connected to a corresponding one transducer element of the N×M array of transducer elements. A shared pulser gate driver and a shared VGA is provided for each row and column. Selection logic includes row select, column select, and per-element bit select.

Wygant, I et al. : "P3H-6 An Integrated Circuit with Transmit Beamforming and Parallel Receive Channels for Real-Time Three-Dimensional Ultrasound Imaging", ULTRASONICS SYMPOSIUM, 2006. IEEE, IEEE, Pl, 2 October 2006, pages 2186-2189, DOI: 10.1109/ULTSYM.2006.551, ISBN: 978-1-4244-0201-4, discloses the design of an integrated circuit (IC) that is flip-chip bonded to a 16 × 16-element CMUT array. The IC provides 16 receive channels which are configured to receive along either of the array diagonals or on any single row of the array.

There is therefore a need for an improved means of handling communication between the ultrasound imaging catheter and the control console.

### SUMMARY

The invention is defined by the claims.

According to examples in the disclosure, there is provided an ultrasound imaging catheter, the ultrasound imaging catheter comprising:
an ultrasound transducer array provided at a distal end of the ultrasound imaging catheter and adapted to transmit and receive ultrasound signals, wherein the ultrasound transducer array comprises a plurality of ultrasound transducers;
a local memory provided at the distal end of the ultrasound imaging catheter adapted to store a plurality of activation patterns, wherein each activation pattern corresponds to a number of transducer elements of the plurality of transducer elements to be activated and a number of transducer elements of the plurality of transducer elements to be deactivated; and
a control unit provided at the distal end of the ultrasound imaging catheter adapted to:
   access the local memory;
   select any one of the plurality of activation patterns; and
   generate a control signal to activate or deactivate the plurality of transducer elements of the transducer array according to the selected activation pattern during an imaging phase of the ultrasound imaging catheter, the imaging phase comprising a transmit period and a receive period.

In this way, the functionality of the front end of the ultrasound imaging catheter may be increased, thereby reducing the amount of communication required between the front end and a back end processing unit, thereby providing for a means of implementing higher frame rates, higher numbers of transducer elements in the transducer array and a higher number of echo return channels.
The ultrasound imaging catheter further comprises:
a plurality of registers, each register comprising a plurality of bits, wherein each bit is associated with an ultrasound transducer of the plurality of ultrasound transducers; and
a local oscillator in communication with the plurality of registers and adapted to receive the control signal generated by the control unit and manipulate the plurality of bits in the plurality of registers to activate and/or deactivate the plurality of transducer elements of the transducer array according to the selected activation pattern.

In this way, an asynchronous communication scheme may be implemented in the ultrasound imaging catheter front end, which would otherwise require a free running clocking signal in order to provide superfluous signal edges in order to initiate the manipulation of the registers.

The local oscillator is adapted to operate outside of the imaging phase of the ultrasound imaging catheter. In this way, potential imaging artifacts due to signal feedthrough or crosstalk from the local oscillator may be avoided.

Alternatively or additionally, the local oscillator is adapted to operate at a frequency that is greater than a bandwidth of the plurality of ultrasound transducers. In this way, imaging artifacts caused by a local oscillator may be reduced, or eliminated.

Alternatively or additionally, the local oscillator is adapted to generate a square wave having a predetermined duty cycle of. The duty cycle is such that components of the local oscillator's frequency spectrum are outside the bandwidth of the plurality of ultrasound transducers. In this way, the harmonics of the local oscillator are made to be outside of the bandwidth of the ultrasound transducers, thereby reducing any imaging artifacts caused by the local oscillator.

In an embodiment, the plurality of registers are arranged into a plurality of register groups, each register group comprising:
a first register, comprising a first register bit;
a second register, comprising a second register bit; and wherein
a transducer element of the plurality of transducer elements is associated with the first register bit, which is adapted to control whether the transducer element is activated or deactivated during the transmit period, and the second register bit, which is adapted to control whether the transducer element is activated or deactivated during the receive period.

By providing individual registers for each function, a greater flexibility in the activation pattern and its application may be achieved.

In a further embodiment, the ultrasound transducer array comprises a plurality of output channels, and wherein each register group further comprises a third register, comprising a third register bit, and wherein the transducer element is further associated with the third register bit, which is adapted to control which of the plurality of output channels a signal received at the transducer element is output to.

In an embodiment, the plurality of register groups are connected in a daisy chain, wherein each register group is connected in series with an adjacent register group.

In this way, the number of connections required in the catheter front end may be reduced, without negatively impacting the functionality of the ultrasound imaging catheter.

In an embodiment, the ultrasound imaging catheter further comprises a signal conditioning unit provided at the distal end of the ultrasound imaging catheter adapted to apply signal conditioning to the received ultrasound signals.

In this way, the signals may be conditioned before being sent to the back end processing unit, thereby reducing imaging artifacts that may be accentuated during transmission and so increasing the accuracy of the final ultrasound image.

In a further embodiment, the signal conditioning unit comprises a low noise amplifier.

In an embodiment, the signal conditioning unit comprises one or more time gain compensation units.

According to examples in accordance with an aspect of the invention, there is provided an ultrasound imaging system, the system comprising:
the ultrasound imaging catheter as described above;
a processing unit in communication with the ultrasound imaging catheter and adapted to generate an ultrasound image based on the received ultrasound signals; and
a display adapted to display the ultrasound image.

According to examples in the disclosure, there is provided a method for controlling an ultrasound imaging catheter comprising an ultrasound transducer array having a plurality of ultrasound transducers, the method comprising:
accessing a local memory provided at the distal end of the ultrasound imaging catheter;
selecting any one of a plurality of activation patterns stored on the local memory, wherein each activation pattern corresponds to a number of transducer elements of the plurality of transducer elements to be activated and a number of transducer elements of the plurality of transducer elements to be deactivated; and
generating a control signal to activate or deactivate the plurality of transducer elements of the transducer array according to the selected activation pattern during an imaging phase of the ultrasound imaging catheter, the imaging phase comprising a transmit period and a receive period.

The method further comprises:
providing the control signal to a local oscillator; and
manipulating a plurality of bits of a plurality of registers, wherein each bit is associated with an ultrasound transducer of the plurality of ultrasound transducer, to activate and/or deactivate the plurality of transducer elements of the transducer array according to the selected activation pattern.

According to examples in the disclosure, there is provided a computer program comprising computer program code means which is adapted, when said computer program is run on a computer, to implement the method described above.

### BRIEF DESCRIPTION OF THE DRAWINGS

For a better understanding of the invention, and to show more clearly how it may be implemented, reference will now be made, by way of example only, to the accompanying drawings, in which:
Fig. 1 shows an ultrasound diagnostic imaging system to explain the general operation;
Fig. 2 shows a schematic representation of an ultrasound imaging catheter;
Fig. 3 shows a schematic representation of the ultrasound imaging catheter of Fig. 2 in greater detail;
Fig. 4 shows an example implementation a part of an ultrasound imaging catheter;
Fig. 5 shows an example register connection scheme;
Fig. 6 shows a schematic representation of the registers with respect to the catheters; and
Fig. 7 shows a method of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound imaging catheter comprising an ultrasound transducer array provided at a distal end of the ultrasound imaging catheter and adapted to transmit and receive ultrasound signals, wherein the ultrasound transducer array comprises a plurality of ultrasound transducers. The ultrasound imaging catheter further includes a local memory provided at the distal end of the ultrasound imaging catheter adapted to store a plurality of activation patterns, wherein each activation pattern corresponds to a number of transducer elements of the plurality of transducer elements to be activated and a number of transducer elements of the plurality of transducer elements to be deactivated. In addition, the ultrasound imaging catheter includes a control unit provided at the distal end of the ultrasound imaging catheter adapted to: access the local memory; select any one of the plurality of activation patterns; and generate a control signal to activate or deactivate the plurality of transducer elements of the transducer array according to the selected activation pattern during an imaging phase of the ultrasound imaging catheter, the imaging phase comprising a transmit period and a receive period.

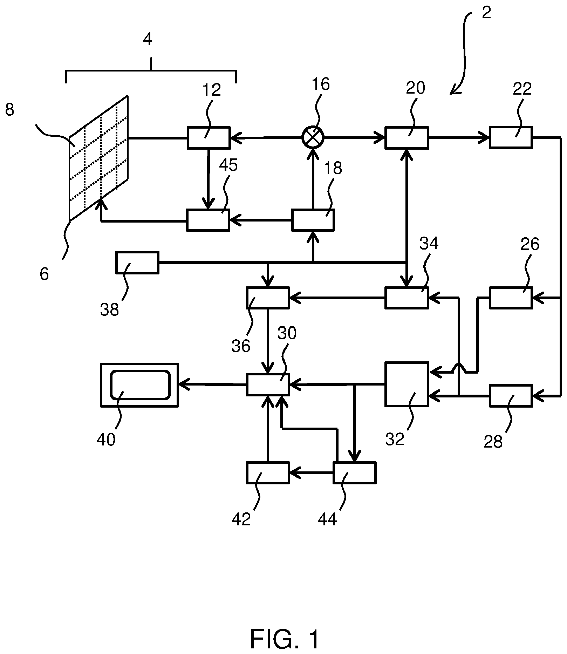

The general operation of an exemplary ultrasound system will first be described, with reference to Fig. 1.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; polymer based transducers; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output integrated into the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

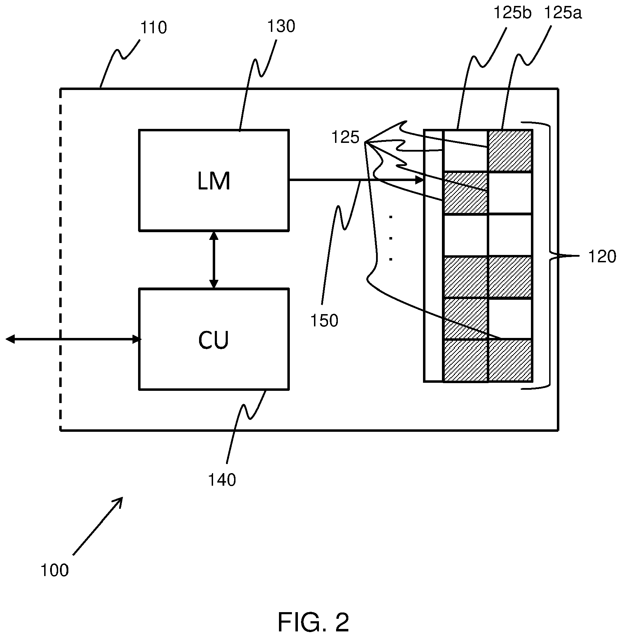

Fig. 2 shows a schematic representation 100 of an ultrasound imaging catheter 110.

The ultrasound imaging catheter 110 comprises an ultrasound transducer array 120 provided at a distal end of the ultrasound imaging catheter comprising a plurality of ultrasound transducers 125.

The ultrasound imaging catheter 110 further comprises a local memory (LM) 130 provided at the distal end of the ultrasound imaging catheter, which is adapted to store a plurality of activation patterns. Each of the plurality of activation patterns corresponds to a number of transducer elements of the plurality of transducer elements to be activated 125a and a number of transducer elements of the plurality of transducer elements to be deactivated 125b during an imaging phase of the ultrasound imaging catheter.

The imaging phase may be regarded as two distinct periods, a transmit period, where ultrasound signals are generated by the ultrasound transducers and transmitted into the subject, and a receive period, wherein echoes from the transmitted ultrasound transducers are received at the ultrasound transducers.

In addition, the ultrasound imaging catheter 110 comprises a control unit (CU) 140 provided at the distal end of the ultrasound imaging catheter adapted to access the local memory and select any one of the plurality of activation patterns stored in the local memory.

The control unit then generates a control signal 150 to activate or deactivate the plurality of transducer elements of the transducer array according to the selected activation pattern. In the example shown in Fig. 2, the LM 130 is manipulated by the CU 140 in order to pass control signal 150 to the transducers, thereby controlling the transducer activity. The CU 140 also acts as the communication link between the circuitry at the distal end of the ultrasound imaging catheter 110 and the rest of the ultrasound system.

By locating the local memory 130 and the control unit 140 at the catheter, rather than at a back end processing unit, the required communication between a back end console and the catheter is reduced.

Put another way, operational control features are added to the catheter, such that it may independently perform at least part of the manipulations in transmit element selection, receive element selection and/or return channel selection. This results in significantly reduced communication requirements between a console and the catheter, allowing higher frame rates, a higher number of ultrasound transducer elements, and higher number of echo return channels to be employed in the system.

The ultrasound imaging catheter may act as the ultrasound probe 4 described above with reference to Fig. 1.

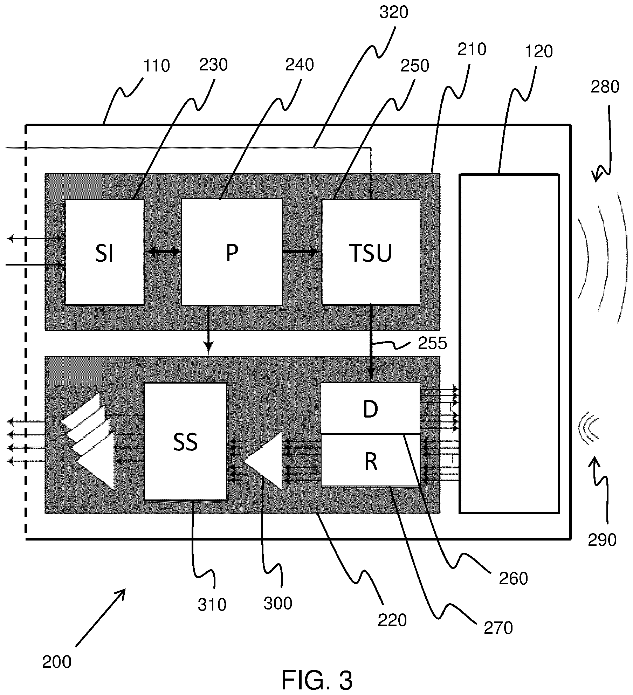

Fig. 3 shows a schematic representation 200 of the ultrasound imaging catheter 110 of Fig. 2 in greater detail.

In the example shown in Fig. 3, the ultrasound imaging catheter 110 is shown as having two component groups, a digital part 210 and an analog part 220.

In the digital part 210 there is a two wire serial interface (SI) 230, which may for example be in communication with a console or any other suitable back end processing unit for controlling the ultrasound imaging catheter 110. Signals may be provided through the serial interface to a parameter control unit (P) 240 adapted to set and read back parameters for controlling the operation of both the analog part 220 and the digital part 210 of the ultrasound imaging catheter.

The digital part 210 further comprises a transducer selection unit (TSU) 250, which may comprise the local memory 130 and control unit 140 described above with reference to Fig. 2.

The transducer selection unit determines which transducer elements of the transducer array 120 should be active during a transmit period, and which transducer elements will be used during a receive period. This is done by way of the control unit accessing the local memory to retrieve one of a plurality of activation patterns.

In the example shown in Fig. 3, the transducer selection unit generates a control signal 255, which is provided to a driver unit (D) 260 and a receiver unit (R) 270. The driver unit activates ultrasound transducers of the transducer array during the transmit period according to the control signal received from the transducer selection unit. Similarly, the receiver unit activates ultrasound transducers of the transducer array during the receive period according to the control signal received from the transducer selection unit.

In other words, the transducers of the ultrasound transducer array are activated according to a control signal generated by a transducer selection unit, which is based on an activation pattern, during the transmit and receive periods of an imaging phase.

Put another way, the transducer elements of a transducer array located at the distal end of an ultrasound imaging catheter are activated based on an activation pattern stored in a local memory also located at said distal end.

During the transmit phase, the ultrasound transducers selected as active according to the activation pattern are operated to generate an ultrasonic pulse, for example when the ultrasound imaging catheter is located inside a subject, such as within a vessel of the subject.

During the receive phase, the ultrasound transducers receive echo signals 290 from the subject's body. The received echo signals may undergo signal conditioning prior to sending the appropriate echo signals to the back end processing system.

In the example shown in Fig. 3, the analog part 220 of the ultrasound imaging catheter 110 comprises a low noise amplifier 300 to condition the incoming received echo signals; however, any other signal conditioning unit may be implemented in the analog part, such as a time gain compensation unit.

The conditioned signals are then passed to a signal selector (SS) 310, which may apply selection criteria to the conditioned signals in order to decide which signals may be sent to a back end processing in order to generate an ultrasound image. The selection criteria may, for example, include a given measure of signal quality, such as a signal to noise ratio. The selection criteria may be adjusted, for example by way of a user input, according to an application of the ultrasound imaging catheter.

When the transducer selection unit receives a signal 320 to initiate a "give next acquisition" instruction, i.e. an instruction to begin a subsequent imaging phase, the transducer selection unit determines the transducers that will be used for the next imaging actions as transmit and receive elements. More specifically, the instruction may cause the control unit to access the local memory in order to select an activation pattern corresponding to the transducers to be activated or deactivated.

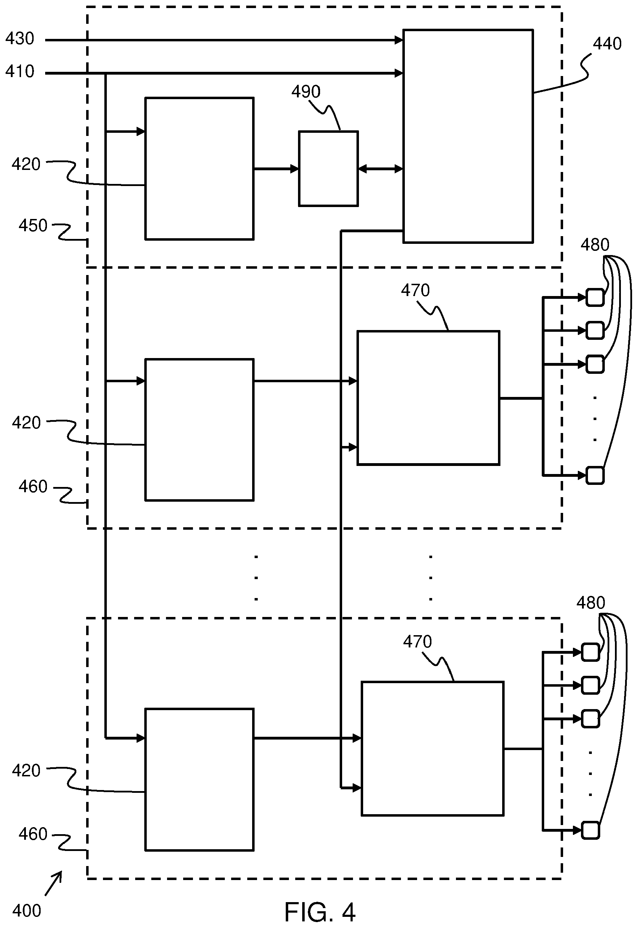

Fig. 4 shows an example implementation 400 of the digital part 210 of the ultrasound imaging catheter of Fig. 3.

In the example shown in Fig. 4, the intelligence level of the transducer selection unit is slightly reduced with respect to the example described with respect to Fig. 3. As a result the communication requirements between the back end processing unit, or console, and the ultrasound imaging catheter may be slightly increased, although the communication requirements are still significantly reduced when compared to a standard ultrasound imaging catheter. However, this example may provide for more flexibility in determining the ultrasound elements to be activated or deactivated during the transmit period and the receive period. Further, this example may also increase the flexibility in the return channel selection for sending the received echo signals to the back end processing system.

The example shown in Fig. 4 operates using two communication mechanisms. The first communication interface 410 is a serial interface for setting and reading back parameters for both the analog and digital parts of the ultrasound imaging catheter as described above with reference to Fig. 3. The serial interface may be connected to an I²C interface 420, which operates based on two signals, a serial clock (SCL) signal and a serial data (SDA) signal.

The second communication interface 430 may comprise a high-speed serial (HS) interface 440, which is a unidirectional equivalent of the "give next acquisition" instruction described above with respect to Fig. 3.

Fig. 4 shows several dies located in the ultrasound imaging catheter; one master die 450 and multiple slave dies 460. The first communication interface 410, which may include an I²C interface 420, may be implemented such that it is the same for all dies. The die type, master die 450 or slave die 460, may be determined by three address pins on the I²C interface unit. If the address pins read "000", the die is considered to be the master die 450. If on the other hand the address pins read between "001" and " 1 1 1", the die is considered to be a slave die 460. In this example, the maximum allowable number of slave dies 460 is seven; however, the number of slave dies may be expanded or reduced according to the application.

The I²C interface unit 420 of the slave dies 460 may be connected to analog circuity, which may comprise components such as the low noise amplifier (LNA) and/or the time gain compensation (TGC) as described above with reference to Fig. 3, as well as connecting a slave unit 470 in each slave die 460. The slave unit 470 is a piece of circuitry that provides an indication for each ultrasound transducer 480 whether it is to active or inactive during transmit, during receive, and on which output channel the received echo signals should be placed.

In the example shown in Fig. 4, each slave die 260 can address a maximum of twenty four individual transducers 280, the information relating to: which transducer elements are active during the transmit periods; which transducer elements are active during the receive periods; and which output channel a received echo signal is provided to being held on three twenty four bit registers. Accordingly, with a maximum of seven slaves, catheters with up to one hundred and sixty eight transducer elements may be constructed according to this implementation.

The first communication interface 410 and the second communication interface 430 are fully asynchronous, means that clock edges in the communication protocols themselves are used to register data. In other words, there is no free running clock in the catheter that can be used to sample the communication interfaces. For several reasons, such as electromagnetic compatibility (EMC) and potential image artefacts, it may be advantageous to not have a free running clock in the catheter during imaging phases. As the number of clock edges in the two communication interfaces is matched with the data being transferred, there are no superfluous edges available for the HS interface 440 to manipulate the information in the registers of the slave units, and so apply the information contained in an application pattern to the ultrasound transducers 480.

Accordingly, the ultrasound imaging catheter may further comprise a local oscillator (LO) 490 in communication with the plurality of registers and adapted to receive the control signal generated by the control unit and manipulate the plurality of bits in the plurality of registers to activate and/or deactivate the plurality of transducer elements of the transducer array according to the selected activation pattern.

The local oscillator 490 may be located on the master die 450 that can be started by the HS unit 440 in order to perform the required manipulations of the registers. The manipulating of registers by the HS unit, and thus running the local oscillator only occurs outside the imaging phases, thereby preventing potential image artefacts due to, for example, local oscillator feedthrough or crosstalk. Another mechanism employed to prevent image artefacts by the local oscillator is to ensure the lowest frequency of the local oscillator is above the bandwidth of the ultrasonic transducers 480. The local oscillator may be adapted to produce a square wave with a given duty cycle in order to bring the harmonics of the local oscillator outside of the bandwidth of the ultrasonic transducers. For example, the duty cycle may be 50%, or 60%, or indeed any suitable duty cycle that ensures that the spectral components of the oscillator are outside of the bandwidth of the plurality of ultrasound transducers.

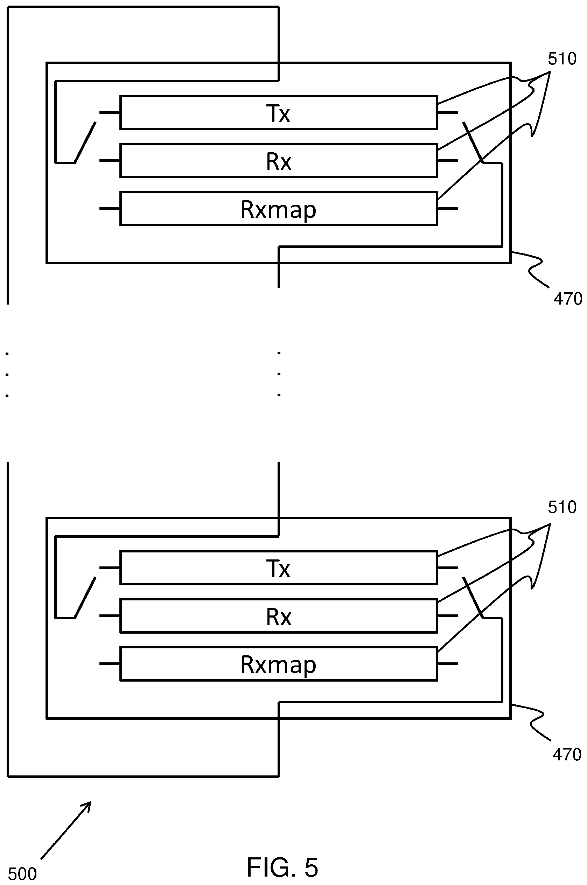

Fig. 5 shows an example 500 of interconnections between registers 510 of different slave units 470 on the slave dies 460. Each register comprises a plurality of bits, wherein each bit is associated with an ultrasound transducer of the plurality of ultrasound transducers in the transducer array of the ultrasound imaging catheter.

In the example shown in Fig. 5, each slave unit 470 comprises three registers 510, which include: a transmit register Tx adapted to control which of the associated transducer elements are activated or deactivated during the transmit period; a receive register Rx adapted to control which of the associated transducer elements are activated or deactivated during the receive period; and an output channel register Rxmap adapted to control which of the plurality of output channels a signal received at the transducer element is output to. In this case, a given transducer element will be associated with one bit from each of the three registers of a given slave unit.

In order to limit the amount of connections required between the slave dies, the registers for transmit (Tx), receive (Rx), and mapping to an output channel (Rxmap) may be connected via a single connection to the next die.

A single connection between dies, also referred to as a daisy chain connection, means that the manipulation of the transmit register, the receive register and the output channel register must be performed sequentially.

To select the appropriate number of transducer elements, there are two methods available: selecting the number of slave dies in the catheter, with a maximum of seven; and selecting the number of elements controlled per die, with a maximum of twenty four.

In the implementation described above, a slave die can control twenty four transducer elements. It is also possible to connect fewer transducer elements to a slave die. The number of transducer elements that are connected into the register daisy chain as shown in Fig. 5 may be set by a parameter in the I²C interface unit 420 described in Fig. 4. The HS unit 440 does not need to be aware of the exact number of transducer elements in the register daisy chain, since the register cells are connected in a daisy chain and no data can may be lost by shift operations. If, for example, a one hundred and fourteen element catheter is required, five slave dies should be used, four of which will control twenty four elements and one of which will control eighteen elements.

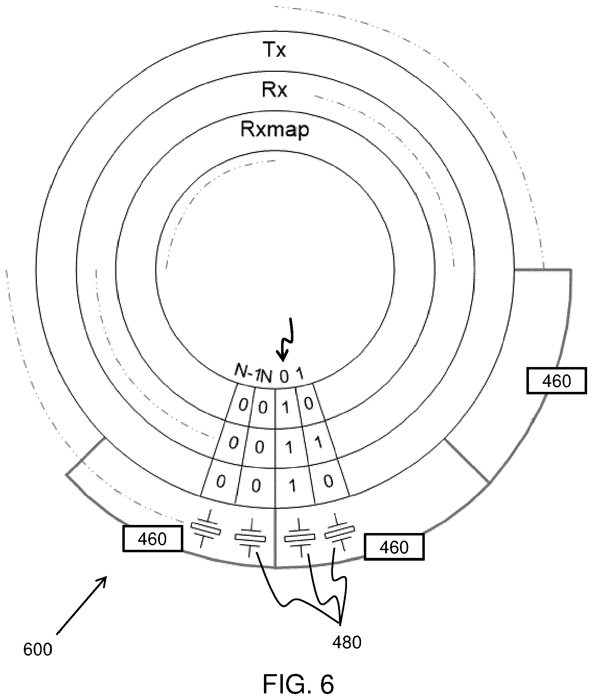

Fig. 6 shows a conceptual representation 600 of the transmit Tx register, the receive Rx register and the output channel Rxmap register in relation to the circular catheter circumference, slave die 460 boundaries and transducer elements 480. Fig. 6 shows some example data, and a meaning for the three register chains.

Looking to transducer element 0, as indicated by the arrow, the registers indicate that this element is to be used for transmitting an ultrasonic pulse, as indicated by the 1 in the transmit Tx register, used for echo reception, as indicated by the 1 in the receive Rx register, and the received echo will be transmitted via channel 1, as indicated by the 1 in the output channel Rxmap register. A 0 in the transmit register or the receive register would indicate that the element would be inactive for the transmit or receive period, respectively. A 0 in the channel output register would indicate that the received echo should be output on channel 0.

It should be noted that the transmit Tx register, the receive Rx register and the output channel Rxmap register, and the combination of the Tx, Rx, and Rxmap bits together, as shown in Fig. 5 and Fig. 6 above may be part of the local memory (LM) 130 as described with reference to Fig. 2.

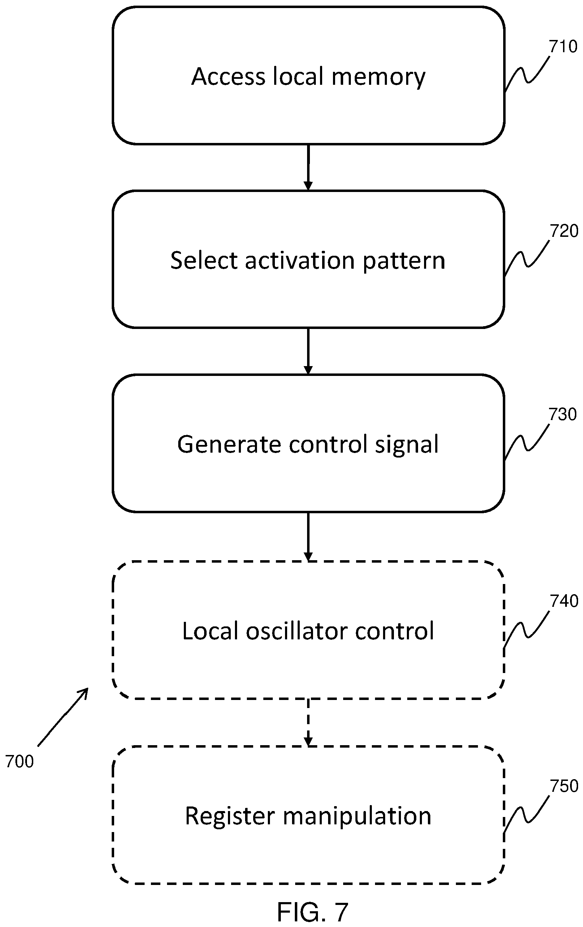

Fig. 7 shows a method 700 for controlling an ultrasound imaging catheter comprising an ultrasound transducer array having a plurality of ultrasound transducers

The method begins in step 710 by accessing a local memory provided at the distal end of the ultrasound imaging catheter;
In step 720, any one of a plurality of activation patterns stored on the local memory is selected, wherein each activation pattern corresponds to a number of transducer elements of the plurality of transducer elements to be activated and a number of transducer elements of the plurality of transducer elements to be deactivated.

In step 730, a control signal to activate or deactivate the plurality of transducer elements of the transducer array according to the selected activation pattern during an imaging phase of the ultrasound imaging catheter is generated, the imaging phase comprising a transmit period and a receive period.

In step 740, the control signal is provided to a local oscillator.

In step 750, a plurality of bits of a plurality of registers is manipulated to activate and/or deactivate the plurality of transducer elements of the transducer array according to the selected activation pattern, wherein each bit is associated with an ultrasound transducer of the plurality of ultrasound transducer.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. An ultrasound imaging catheter (110), comprising:
an ultrasound transducer array (120) provided at a distal end of the ultrasound imaging catheter and adapted to transmit and receive ultrasound signals, wherein the ultrasound transducer array comprises a plurality of ultrasound transducer elements (125);
a local memory (130) provided at the distal end of the ultrasound imaging catheter adapted to store a plurality of activation patterns, wherein each activation pattern corresponds to a number of transducer elements of the plurality of transducer elements to be activated and a number of transducer elements of the plurality of transducer elements to be deactivated;
a control unit (140) provided at the distal end of the ultrasound imaging catheter adapted to:
access the local memory;
select any one of the plurality of activation patterns;
generate a control signal (150) to activate or deactivate the plurality of transducer elements of the transducer array according to the selected activation pattern during an imaging phase of the ultrasound imaging catheter, the imaging phase comprising a transmit period and a receive period;
wherein the ultrasound imaging catheter further comprises:
a plurality of registers (510), each register comprising a plurality of bits, wherein each bit is associated with an ultrasound transducer element of the plurality of ultrasound transducer elements;
a local oscillator (490) in communication with the plurality of registers and adapted to receive the control signal generated by the control unit and manipulate the plurality of bits in the plurality of registers to activate and/or deactivate the plurality of transducer elements of the transducer array according to the selected activation pattern; **characterized in** at least one of:
the local oscillator being adapted to operate outside of the imaging phase of the ultrasound imaging catheter;
the local oscillator being adapted to operate at a frequency that is greater than a bandwidth of the plurality of ultrasound transducer elements;
the local oscillator being adapted to generate a square wave having a given duty cycle, wherein the given duty cycle is such that components of the local oscillator's frequency spectrum are outside the bandwidth of the plurality of ultrasound transducer elements.

2. The ultrasound imaging catheter of claim 1, wherein the plurality of registers are arranged into a plurality of register groups, each register group comprising:
a first register, comprising a first register bit;
a second register, comprising a second register bit; and wherein
a transducer element of the plurality of transducer elements is associated with the first register bit, which is adapted to control whether the transducer element is activated or deactivated during the transmit period, and the second register bit, which is adapted to control whether the transducer element is activated or deactivated during the receive period.

3. The ultrasound imaging catheter of claim 2, wherein the ultrasound transducer array comprises a plurality of output channels, and wherein each register group further comprises a third register, comprising a third register bit, and wherein the transducer element is further associated with the third register bit, which is adapted to control which of the plurality of output channels a signal received at the transducer element is output to.

4. The ultrasound imaging catheter of claim 2 or 3, wherein the plurality of register groups are connected in a daisy chain, wherein each register group is connected in series with an adjacent register group.

5. The ultrasound imaging catheter of any of claims 1 to 4, wherein the ultrasound imaging catheter further comprises a signal conditioning unit provided at the distal end of the ultrasound imaging catheter adapted to apply signal conditioning to the received ultrasound signals.

6. The ultrasound imaging catheter of claim 5, wherein the signal conditioning unit comprises a low noise amplifier.

7. The ultrasound imaging catheter of claim 5 or 6, wherein the signal conditioning unit comprises one or more time gain compensation units.

8. An ultrasound imaging system, the system comprising:
the ultrasound imaging catheter of any of claims 1 to 7;
a processing unit in communication with the ultrasound imaging catheter and adapted to generate an ultrasound image based on the received ultrasound signals; and
a display adapted to display the ultrasound image.

9. A method (700) of controlling an ultrasound imaging catheter (110) comprising an ultrasound transducer array (120) having a plurality of ultrasound transducer elements (125), the method comprising:
accessing (710) a local memory (130) provided at the distal end of the ultrasound imaging catheter;
selecting (720) any one of a plurality of activation patterns stored on the local memory, wherein each activation pattern corresponds to a number of transducer elements of the plurality of transducer elements to be activated and a number of transducer elements of the plurality of transducer elements to be deactivated;
generating (730) a control signal (150) to activate or deactivate the plurality of transducer elements of the transducer array according to the selected activation pattern during an imaging phase of the ultrasound imaging catheter, the imaging phase comprising a transmit period and a receive period;
providing (740) the control signal to a local oscillator;
manipulating (750) a plurality of bits of a plurality of registers, wherein each bit is associated with an ultrasound transducer element of the plurality of ultrasound transducer elements, to activate and/or deactivate the plurality of transducer elements of the transducer array according to the selected activation pattern;
**characterized by**:
operating the local oscillator outside of the imaging phase of the ultrasound imaging catheter;
operating the local oscillator at a frequency that is greater than a bandwidth of the plurality of ultrasound transducer elements;
generating by the local oscillator a square wave having a given duty cycle, wherein the given duty cycle is such that components of the local oscillator's frequency spectrum are outside the bandwidth of the plurality of ultrasound transducer elements.

10. A computer program comprising computer program code means which is adapted, when said computer program is run on a control unit according to claim 1, to implement the method of claim 9.

## Patentansprüche

1. Ultraschallbildgebungskatheter (110), umfassend:
ein Ultraschallwandlerarray (120), das an einem distalen Ende des Ultraschallbildgebungskatheters bereitgestellt ist und angepasst ist, um Ultraschallsignale zu senden und zu empfangen, wobei das Ultraschallwandlerarray eine Vielzahl von Ultraschallwandlerelementen (125) umfasst;
einen lokalen Speicher (130), der am distalen Ende des Ultraschallbildgebungskatheters bereitgestellt ist und angepasst ist, um eine Vielzahl von Aktivierungsmustern zu speichern, wobei jedes Aktivierungsmuster einer Anzahl von Wandlerelementen aus der Vielzahl von Wandlerelementen, die zu aktivieren sind, und einer Anzahl von Wandlerelementen aus der Vielzahl von Wandlerelementen, die zu deaktivieren sind, entspricht;
eine Steuereinheit (140), die am distalen Ende des Ultraschallbildgebungskatheters bereitgestellt ist und angepasst ist, um:
auf den lokalen Speicher zuzugreifen;
eines der Vielzahl von Aktivierungsmustern auszuwählen;
ein Steuersignal (150) zu erzeugen, um die Vielzahl von Wandlerelementen des Wandlerarrays gemäß dem ausgewählten Aktivierungsmuster während einer Bildgebungsphase des Ultraschallbildgebungskatheters zu aktivieren oder deaktivieren, wobei die Bildgebungsphase eine Sendeperiode und eine Empfangsperiode umfasst;
wobei der Ultraschallbildgebungskatheter weiter umfasst:
eine Vielzahl von Registern (510), wobei jedes Register eine Vielzahl von Bits umfasst, wobei jedes Bit mit einem Ultraschallwandlerelement der Vielzahl von Ultraschallwandlerelementen verknüpft ist;
einen lokalen Oszillator (490), der mit der Vielzahl von Registern in Kommunikation steht und angepasst ist, um das von der Steuereinheit erzeugte Steuersignal zu empfangen und die Vielzahl von Bits in der Vielzahl von Registern zu manipulieren, um die Vielzahl von Wandlerelementen des Wandlerarrays gemäß dem ausgewählten Aktivierungsmuster zu aktivieren und/oder deaktivieren; **dadurch gekennzeichnet, dass** mindestens eines von:
dem lokalen Oszillator angepasst ist, um außerhalb der Bildgebungsphase des Ultraschallbildgebungskatheters zu arbeiten;
dem lokalen Oszillator angepasst ist, um bei einer Frequenz zu arbeiten, die größer ist als eine Bandbreite der Vielzahl von Ultraschallwandlerelementen;
dem lokalen Oszillator angepasst ist, um eine Rechteckwelle zu erzeugen, die einen gegebenen Arbeitszyklus aufweist, wobei der gegebene Arbeitszyklus derart ist, dass Komponenten des Frequenzspektrums des lokalen Oszillators außerhalb der Bandbreite der Vielzahl von Ultraschallwandlerelementen liegen.

2. Ultraschallbildgebungskatheter nach Anspruch 1, wobei die Vielzahl von Registern in einer Vielzahl von Registergruppen angeordnet ist, wobei jede Registergruppe umfasst:
ein erstes Register, das ein erstes Registerbit umfasst;
ein zweites Register, das ein zweites Registerbit umfasst; und wobei
ein Wandlerelement der Vielzahl von Wandlerelementen mit dem ersten Registerbit verknüpft ist, das angepasst ist, um zu steuern, ob das Wandlerelement während der Sendeperiode aktiviert oder deaktiviert ist, und mit dem zweiten Registerbit, das angepasst ist, um zu steuern, ob das Wandlerelement während der Empfangsperiode aktiviert oder deaktiviert ist.

3. Ultraschallbildgebungskatheter nach Anspruch 2, wobei das Ultraschallwandlerarray eine Vielzahl von Ausgangskanälen umfasst, und wobei jede Registergruppe weiter ein drittes Register umfasst, das ein drittes Registerbit umfasst, und wobei das Wandlerelement weiter mit dem dritten Registerbit verknüpft ist, das angepasst ist, um zu steuern, an welchen der Vielzahl von Ausgangskanälen ein am Wandlerelement empfangenes Signal auszugeben ist.

4. Ultraschallbildgebungskatheter nach Anspruch 2 oder 3,
wobei die Vielzahl von Registergruppen in einer Daisy-Chain verbunden sind, wobei jede Registergruppe in Reihe mit einer benachbarten Registergruppe verbunden ist.

5. Ultraschallbildgebungskatheter nach einem der Ansprüche 1 bis 4, wobei der Ultraschallbildgebungskatheter weiter eine Signalaufbereitungseinheit umfasst, die am distalen Ende des Ultraschallbildgebungskatheters bereitgestellt ist und angepasst ist, um eine Signalaufbereitung auf die empfangenen Ultraschallsignale anzuwenden.

6. Ultraschallbildgebungskatheter nach Anspruch 5, wobei die Signalaufbereitungseinheit einen rauscharmen Verstärker umfasst.

7. Ultraschallbildgebungskatheter nach Anspruch 5 oder 6, wobei die Signalaufbereitungseinheit eine oder mehrere Einheiten zur Zeitverstärkungskompensation umfasst.

8. Ultraschallbildgebungssystem, wobei das System umfasst:
den Ultraschallbildgebungskatheter nach einem der Ansprüche 1 bis 7;
eine Verarbeitungseinheit, die mit dem Ultraschallbildgebungskatheter in Kommunikation steht und angepasst ist, um basierend auf den empfangenen Ultraschallsignalen ein Ultraschallbild zu erzeugen; und
eine Anzeige, die angepasst ist, um das Ultraschallbild anzuzeigen.

9. Verfahren (700) zum Steuern eines Ultraschallbildgebungskatheters (110), der ein Ultraschallwandlerarray (120) umfasst, das eine Vielzahl von Ultraschallwandlerelementen (125) aufweist, wobei das Verfahren umfasst:
Zugreifen (710) auf einen lokalen Speicher (130), der am distalen Ende des Ultraschallbildgebungskatheters bereitgestellt ist;
Auswählen (720) eines beliebigen einer Vielzahl von Aktivierungsmustern, die im lokalen Speicher gespeichert sind, wobei jedes Aktivierungsmuster einer Anzahl von Wandlerelementen aus der Vielzahl von Wandlerelementen, die zu aktivieren sind, und einer Anzahl von Wandlerelementen aus der Vielzahl von Wandlerelementen, die zu deaktivieren sind, entspricht;
Erzeugen (730) eines Steuersignals (150), um die Vielzahl von Wandlerelementen des Wandlerarrays gemäß dem ausgewählten Aktivierungsmuster während einer Bildgebungsphase des Ultraschallbildgebungskatheters zu aktivieren oder deaktivieren, wobei die Bildgebungsphase eine Sendeperiode und eine Empfangsperiode umfasst;
Bereitstellen (740) des Steuersignals für einen lokalen Oszillator;
Manipulieren (750) einer Vielzahl von Bits einer Vielzahl von Registern, wobei jedes Bit mit einem Ultraschallwandlerelement der Vielzahl von Ultraschallwandlerelementen verknüpft ist, um die Vielzahl von Wandlerelementen des Wandlerarrays gemäß dem ausgewählten Aktivierungsmuster zu aktivieren und/oder zu deaktivieren; **gekennzeichnet durch**:
Betreiben des lokalen Oszillators außerhalb der Bildgebungsphase des Ultraschallbildgebungskatheters;
Betreiben des lokalen Oszillators bei einer Frequenz, die größer ist als eine Bandbreite der Vielzahl von Ultraschallwandlerelementen;
Erzeugen durch den lokalen Oszillator einer Rechteckwelle, die einen gegebenen Arbeitszyklus aufweist, wobei der gegebene Arbeitszyklus derart ist, dass Komponenten des Frequenzspektrums des lokalen Oszillators außerhalb der Bandbreite der Vielzahl von Ultraschallwandlerelementen liegen.

10. Computerprogramm, umfassend Computerprogrammcodemittel, die angepasst sind, wenn das Computerprogramm auf einer Steuereinheit nach Anspruch 1 läuft, das Verfahren nach Anspruch 9 zu implementieren.

## Revendications

1. Cathéter d'imagerie ultrasonore (110), comprenant :
un réseau de transducteurs à ultrasons (120) disposé sur une extrémité distale du cathéter d'imagerie ultrasonore et conçu pour émettre et recevoir des signaux ultrasonores, dans lequel le réseau de transducteurs à ultrasons comprend une pluralité d'éléments transducteurs à ultrasons (125) ;
une mémoire locale (130) disposée sur l'extrémité distale du cathéter d'imagerie ultrasonore conçue pour stocker une pluralité de modèles d'activation, dans lequel chaque modèle d'activation correspond à un certain nombre d'éléments transducteurs de la pluralité d'éléments transducteurs à activer et à un certain nombre d'éléments transducteurs de la pluralité d'éléments transducteurs à désactiver ;
une unité de commande (140) disposée sur l'extrémité distale du cathéter d'imagerie ultrasonore conçue pour :
accéder à la mémoire locale ;
sélectionner l'un quelconque de la pluralité de modèles d'activation ;
générer un signal de commande (150) pour activer ou désactiver la pluralité d'éléments transducteurs du réseau de transducteurs selon le modèle d'activation sélectionné pendant une phase d'imagerie du cathéter d'imagerie ultrasonore, la phase d'imagerie comprenant une période d'émission et une période de réception ;
dans lequel le cathéter d'imagerie ultrasonore comprend en outre :
une pluralité de registres (510), chaque registre comprenant une pluralité de bits, dans lequel chaque bit est associé à un élément transducteur à ultrasons de la pluralité d'éléments transducteurs à ultrasons ;
un oscillateur local (490) en communication avec la pluralité de registres et conçu pour recevoir le signal de commande généré par l'unité de commande et manipuler la pluralité de bits dans la pluralité de registres pour activer et/ou désactiver la pluralité d'éléments transducteurs du réseau de transducteurs selon le modèle d'activation sélectionné ; **caractérisé par** au moins l'un de :
l'oscillateur local est conçu pour fonctionner en dehors de la phase d'imagerie du cathéter d'imagerie ultrasonore ;
l'oscillateur local est conçu pour fonctionner à une fréquence qui est supérieure à une bande passante de la pluralité d'éléments transducteurs à ultrasons ;
l'oscillateur local est conçu pour générer une onde carrée présentant un facteur d'utilisation donné, dans lequel le facteur d'utilisation donné est tel que les composantes du spectre de fréquence de l'oscillateur local sont en dehors de la bande passante de la pluralité d'éléments transducteurs à ultrasons.

2. Cathéter d'imagerie ultrasonore selon la revendication 1, dans lequel la pluralité de registres sont agencés en une pluralité de groupes de registres, chaque groupe de registres comprenant :
un premier registre, comprenant un premier bit de registre ;
un deuxième registre, comprenant un deuxième bit de registre ; et dans lequel
un élément transducteur de la pluralité d'éléments transducteurs est associé au premier bit de registre, qui est conçu pour commander si l'élément transducteur est activé ou désactivé pendant la période d'émission, et au deuxième bit de registre qui est conçu pour commander si l'élément transducteur est activé ou désactivé pendant la période de réception.

3. Cathéter d'imagerie ultrasonore selon la revendication 2, dans lequel le réseau de transducteurs à ultrasons comprend une pluralité de canaux de sortie et dans lequel chaque groupe de registres comprend en outre un troisième registre, comprenant un troisième bit de registre, et dans lequel l'élément transducteur est en outre associé au troisième bit de registre qui est conçu pour commander vers quel canal de sortie de la pluralité de canaux de sortie est envoyé un signal reçu au niveau de l'élément transducteur.

4. Cathéter d'imagerie ultrasonore selon la revendication 2 ou 3,
dans lequel la pluralité de groupes de registres sont connectés en chaîne, dans lequel chaque groupe de registres est connecté en série avec un groupe de registres adjacent.

5. Cathéter d'imagerie ultrasonore selon l'une quelconque des revendications 1 à 4, dans lequel le cathéter d'imagerie ultrasonore comprend en outre une unité de conditionnement de signal disposée sur l'extrémité distale du cathéter d'imagerie ultrasonore, conçue pour appliquer un conditionnement de signal aux signaux ultrasonores reçus.

6. Cathéter d'imagerie ultrasonore selon la revendication 5, dans lequel l'unité de conditionnement de signal comprend un amplificateur à faible bruit.

7. Cathéter d'imagerie ultrasonore selon la revendication 5 ou 6, dans lequel l'unité de conditionnement de signal comprend une ou plusieurs unités de compensation de gain de temps.

8. Système d'imagerie ultrasonore, le système comprenant :
le cathéter d'imagerie ultrasonore selon l'une quelconque des revendications 1 à 7 ;
une unité de traitement en communication avec le cathéter d'imagerie ultrasonore et conçue pour générer une image ultrasonore sur la base des signaux ultrasonores reçus ; et
un écran conçu pour afficher l'image ultrasonore.

9. Procédé (700) de commande d'un cathéter d'imagerie ultrasonore (110) comprenant un réseau de transducteurs à ultrasons (120) présentant une pluralité d'éléments transducteurs à ultrasons (125), le procédé comprenant :
l'accès (710) à une mémoire locale (130) disposée sur l'extrémité distale du cathéter d'imagerie ultrasonore ;
la sélection (720) de l'un quelconque d'une pluralité de modèles d'activation stockés dans la mémoire locale, dans lequel chaque modèle d'activation correspond à un certain nombre d'éléments transducteurs de la pluralité d'éléments transducteurs à activer et à un certain nombre d'éléments transducteurs de la pluralité d'éléments transducteurs à désactiver ;
la génération (730) d'un signal de commande (150) pour activer ou désactiver la pluralité d'éléments transducteurs du réseau de transducteurs selon le modèle d'activation sélectionné pendant une phase d'imagerie du cathéter d'imagerie ultrasonore, la phase d'imagerie comprenant une période d'émission et une période de réception ;
la fourniture (740) du signal de commande à un oscillateur local ;
la manipulation (750) d'une pluralité de bits d'une pluralité de registres, dans lequel chaque bit est associé à un élément transducteur à ultrasons de la pluralité d'éléments transducteurs à ultrasons, pour activer et/ou désactiver la pluralité d'éléments transducteurs du réseau de transducteurs selon le modèle d'activation sélectionné ; **caractérisé par** :
le fonctionnement de l'oscillateur local en dehors de la phase d'imagerie du cathéter d'imagerie ultrasonore ;
le fonctionnement de l'oscillateur local à une fréquence qui est supérieure à une bande passante de la pluralité d'éléments transducteurs à ultrasons ;
la génération, par l'oscillateur local, d'une onde carrée présentant un facteur d'utilisation donné, dans lequel le facteur d'utilisation donné est tel que les composantes du spectre de fréquence de l'oscillateur local sont en dehors de la bande passante de la pluralité d'éléments transducteurs à ultrasons.

10. Programme informatique comprenant un moyen de code de programme informatique qui est conçu, lorsque ledit programme informatique est exécuté sur une unité de commande selon la revendication 1, pour mettre en oeuvre le procédé de la revendication 9.
